# EUROPEAN PATENT APPLICATION

(11) **EP 3 714 778 A1**
(43) Date of publication of application: **30.09.2020**
(21) Application number: 19166335.0
(22) Date of filing: 29.03.2019
(51) Int. Cl.: A61B 5/0205, A61B 5/00, C08L 53/02, A61B 5/024, A61B 5/053, A61B 5/1455

(54) **WEARABLE SENSOR DEVICE**

(71) Applicant: Koninklijke Philips N.V., 5656 AG Eindhoven (NL)
(72) Inventor: Ouwerkerk, Martin, 5656 AE Eindhoven (NL); Berben, Edward Theodorus Maria, 5656 AE Eindhoven (NL)
(74) Representative: de Haan, Poul Erik

(57) **Abstract**

The present invention relates to a wearable sensor device (10, 20) configured to be worn by a user for sensing a parameter of the user. To prevent or at least reduce shifts of the wearable sensor device on the user's skin while the wearable sensor device is worn by the user and thus provide an improved accuracy and/or reliability of the measurement, the wearable sensor device comprises a holding element (11, 15), at least one sensor element (13, 31) arranged in or on the holding element for contacting skin of the user and sensing a parameter of the user, and a ring-shaped anti-slip element (14) arranged on the holding element around the at least one sensor element, the anti-slip element protruding from the holding element and being made of styrene ethylene butadiene styrene.

## Description

### FIELD OF THE INVENTION

The present invention relates to a wearable sensor device configured to be worn by a user for sensing a parameter of the user, for instance for measuring skin conductance, blood oxygenation (SpO2), pulse rate or other parameter of a user wearing the wearable sensor device.

### BACKGROUND OF THE INVENTION

US 9706942 discloses a wearable device wearable by a user for measuring skin conductance of the user. The disclosed wearable device comprises at least two skin conductance electrodes for contacting skin of the user and an elastic material portion which surrounds the skin conductance electrodes and forms a material surface. The elastic material of the elastic material portion is non-permeable for gaseous and liquid substances.

Skin conductance measurements performed by such a wearable device depend on sweat for an electrical connection between electronics and sweat glands to be able to monitor sweat gland activity which in turn is a measure of sympathetic nervous system activity. When worn on a location prone to be subjected to movement, such as the wrist, a location shift of the wearable device can occur shifting it from a moist spot to a dry spot. Such a shift diminishes or even nullifies the amount of sweat glands monitored by the wearable device, having a negative impact on signal to noise ratio.

Similar problems may arise with other wearable devices for measuring other parameters, e.g. pulse rate or SpO2, where shifts of the wearable device caused e.g. by movements of the user, may cause a reduction of the accuracy and/or reliability of the measured parameter since the measurement spot on the user's skin may change.

### SUMMARY OF THE INVENTION

It is an object of the present invention to provide a wearable sensor device that prevents or at least reduces shifts of the wearable sensor device on the user's skin while the wearable sensor device is worn by the user and thus provides an improved accuracy and/or reliability of the measurement.

According to the present invention a wearable sensor device configured to be worn by a user for sensing a parameter of the user is presented, the wearable sensor device comprising:
- a holding element,
- at least one sensor element arranged in or on the holding element for contacting skin of the user and sensing a parameter of the user, and
- a ring-shaped anti-slip element arranged on the holding element around the at least one sensor element, the anti-slip element protruding from the holding element and being made of styrene ethylene butadiene styrene.

Preferred embodiments of the invention are defined in the dependent claims.

The present invention is based on the idea to use a (closed or partly open) ring of styrene ethylene butadiene styrene (SEBS) as an anti-slip measure preventing device location shifts. SEBS has the advantage that it is skin-friendly.

The flexible material ring, i.e. the ring-shaped anti-slip element, particularly if it formed is a closed ring, may thus also serve as a container of the moist micro-climate by prevention of evaporation from the space within the ring between the skin and the holding element, in or on which the at least one sensor element is arranged. Instead of forming the anti-slip element as a closed ring, in another embodiment the ring-shaped anti-slip element is formed as open ring comprising two or more ring sections with openings in between. This is particularly useful, if it is not desired to maintain a moist micro-climate within the ring, but to release any fluid from this area if this may influence the measurement.

In another embodiment the at least one sensor and the ring-shaped anti-slip element protrude from the holding element, wherein the ring-shaped anti-slip element protrudes from the holding element to a larger extent than the at least one sensor. This ensures the desired anti-slip effect and, if the ring is closed, the desired micro-climate effect.

The styrene ethylene butadiene styrene preferably has a low hardness, in particular a hardness below 30 Shore A. A range of 26 to 30 Shore A hardness may be preferred. The material should be elastic and not exhibiting easy plastic deformation. An easy elastically deformable material adapts to muscle tendon movements in the wrist and thus maintains a maximal contact area at all times. Too soft materials may exhibit easy plastic deformation, and too hard materials may lose contact with the skin upon flexing of muscle tendons.

The holding element may be a back plate of a housing or part of a fixation element for fixing the wearable sensor device to a body part of the user. For instance, the device may be configured as wrist-worn device, like a watch, so that the holding element is the back plate (facing the skin) of the housing of the wrist-worn device. In other embodiments the device may be configured to be mounted to other body parts, e.g. to the user's chest by use of a belt, in which case the surface of a particular region of the belt may be the holding element. Still further, the device may be embedded into a virtual reality headset so that the headset may comprise built-in skin conductance sensors and/or PPG heart rate sensors. The sensors are arranged such that they are contact the forehead of the user.

In another element the wearable sensor device may further comprise a fixation element for fixing the wearable sensor device to a body part of the user. The fixation element may e.g. be a wrist band or belt. The ring-shaped anti-slip element thus does not provide the fixation of the body part of the user, but the fixation is (solely) provided by the fixation element, and the ring-shaped anti-slip element just provides that the device does not slip.

In an embodiment, the device may comprise at least two skin conductance electrodes and/or a plethysmography sensor including a light source and a photo detector and/or an infrared thermometer. The device may thus be used to measure skin conductance and/or vital signs like SpO2 or pulse rate and/or body temperature.

In another embodiment the ring-shaped anti-slip element is arranged such that an air pocket is sealed off in the vicinity of the at least one sensor, when the at least one sensor contacts the skin of the user. A hermetic sealing of a small area surrounding the at least one sensor from the outside atmosphere is thus achieved, which may improve the measurement even further.

In an embodiment the at least one sensor protrudes between 50 to 2000 micrometer from the holding element, in particular between 100 and 700 micrometer. These protrusion distances have shown to be particularly suitable to seal off the air pocket.

In another embodiment, the device may further comprise a processing unit configured to process the sensed parameter of the user. Hence, the evaluation of the measurement may be made in the device itself.

The processing unit (and optionally further electronic elements) may at least partly be arranged in a housing portion. The housing portion may be made of a non-elastic material to provide for a more robust device. Further electronic elements may e.g. be a voltage generator for applying a voltage, e.g. between at least two skin conductance electrodes or to a light source (e.g. an LED of a photoplethysmography sensor) and/or a sensing unit for sensing a current, e.g. between the at least two skin conductance electrodes. Further, a signal output may be provided, e.g. a (wireless) communication unit (e.g. using Bluetooth or WiFi), to transmit the measurement or a processed signal to an external unit (e.g. a computer or smartphone) and/or a display.

The styrene ethylene butadiene styrene may have an elasticity that is sufficient to follow the shape of the wrist of the user. In this way, the device can be put around or onto the wrist of the user. Measuring e.g. skin conductance or another vital sign on the wrist using a wristband is an unobtrusive way of measurement. The at least one sensor is preferably arranged so as to contact the dorsal (and/or volar) side of the wrist. Placement at the dorsal side makes it possible to build the electrodes e.g. in the back plate of a smartwatch. Emotion induced sweating is less at this location compared to the volar side of the wrist. Placement at the volar side of the wrist may have the advantage that there is normally not a lot of hair that could influence the measurement of the skin conductance or other parameters

### BRIEF DESCRIPTION OF THE DRAWINGS

These and other aspects of the invention will be apparent from and elucidated with reference to the embodiment(s) described hereinafter. In the following drawings
Fig. 1 shows a diagram of a skin conductance trace and an accelerometer signal measured simultaneously,
Fig. 2 shows a cross-sectional view (Fig. 2A) and a bottom view (Fig. 2B) of the first embodiment of the wearable sensor device according to the present invention,
Fig. 3 shows the chemical formula of SEBS,
Fig. 4 shows a perspective view of a second embodiment of a wearable sensor device according to the present invention,
Fig. 5 shows a cross-sectional view and a bottom view of a third embodiment of a wearable sensor device according to the present invention,
Fig. 6 shows a bottom view (Fig. 6A) and two cross-sectional views (Fig. 6B and Fig. 6C) of a fourth embodiment of a wearable sensor device according to the present invention, and
Fig. 7 shows two cross-sectional views (Fig. 7A and Fig. 7B) of a fifth embodiment of a wearable sensor device according to the present invention.

### DETAILED DESCRIPTION OF THE EMBODIMENTS

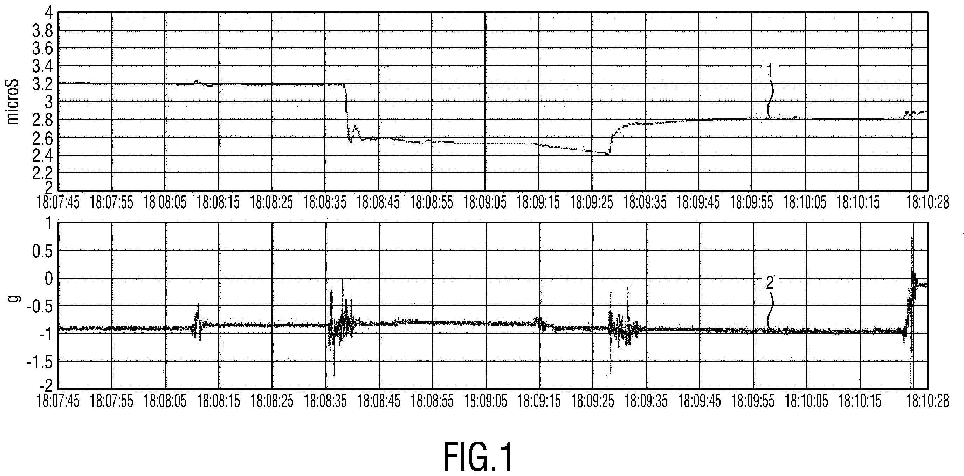

Fig. 1 shows a diagram of a skin conductance trace 1 and an accelerometer signal 2, both measured simultaneously by a wearable sensor device worn at a user's left wrist. It can be seen from these signals that a sudden drop in skin conductance is caused by a shift of the wearable sensor device (indicated by signal changes in the accelerometer signal 2) and that a partial recovery of the skin conductance is caused by shifting back the wearable sensor.

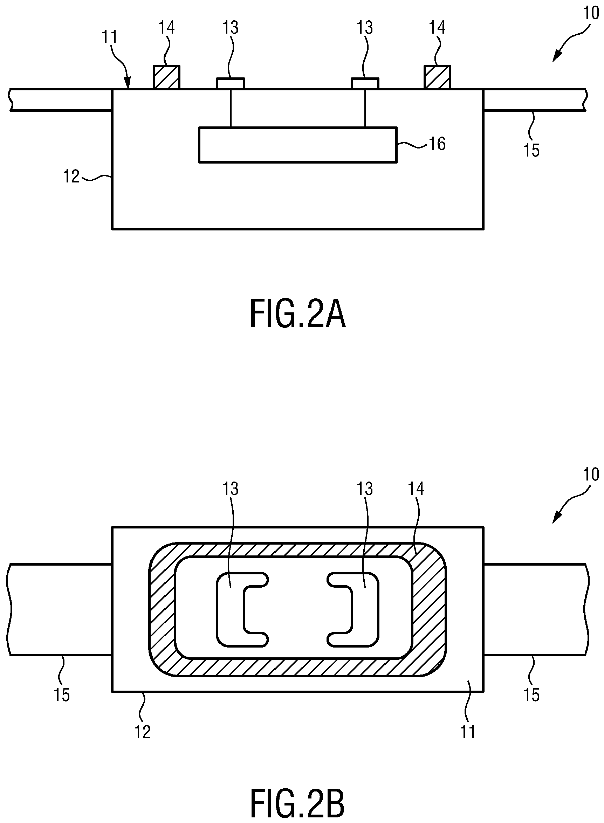

Fig. 2 shows a cross-sectional view (Fig. 2A) and a bottom view (Fig. 2B) of a first embodiment of the wearable sensor device 10 according to the present invention. In this exemplary embodiment, the wearable sensor device 10 is configured to measure skin conductance of a user.

The wearable sensor device 10 comprises a holding element 11, in this embodiment the back plate of a housing 12, which may be formed like a wrist watch and can thus be worn at the wrist by a user.

The wearable sensor device 10 further comprises two skin conductance electrodes 13 for contacting skin of the user and sensing a skin conductance of the user. The two skin conductance electrodes 13 are arranged on the holding element 11 so that they protrude from the holding element 11. The holding element 11 may be implemented by different elements as will be explained below by reference to different exemplary embodiments.

The wearable sensor device 10 further comprises a ring-shaped anti-slip element 14 arranged on the holding element 11 around the two skin conductance electrodes 13. The anti-slip element 14 protrudes from the holding element 11 and is made of styrene ethylene butadiene styrene (SEBS). In this embodiment the anti-slip element 14 is formed as a closed ring and thus circumferentially encloses the electrodes 13. The anti-slip element 14 is e.g. glued to the holding element 11 using a suitable glue, e.g. a skin friendly glue.

For arranging the wearable sensor device 10 to the desired body part, here the wrist, a fixation element 15, e.g. a wristband or strap, is provided.

Hence, in this embodiment a ring of SEBS as an anti-slip measure preventing device location shifts is fitted to the holding element 11, i.e. the back plate of the housing 12 in this exemplary embodiment. A variant of SEBS with a low hardness is preferred, particularly a variant which can deform already at forces present between a wearable sensor device 10 worn at the wrist, with the fixation element 15 tightened to such an extent that the device 10 is comfortable to wear, in combination that dangling or loss of skin contact is minimized.

SEBS is non-permeable for gaseous and liquid substances. For example, the elastic material is non-permeable for water, water vapor and/or air. In this way a fluid film is generated between the skin and the electrodes when the wearable sensor device is worn by the user. The fluid film is made of (human) sweat of the user from sweat glands. It is achieved that the fluid loss through re-uptake of the skin (or stratum corneum) plus evaporation of fluid to outside ambient is lower than the fluid generation through the sweat glands. In particular, as SEBS is non-permeable for air and water, the elastic material is non-breathable and non-water-permeable. In this way, a stable microclimate in the immediate surroundings or vicinity of the skin conductance electrodes 13 can be achieved. Thus, the use of the anti-slip element 14 surrounding the skin conductance electrodes 13 creates a moist microclimate, causing the skin conductance measurement or sensor data to stay within a measurable range for basically all users for basically all of the time. A high skin conductance level for all skin types and all environments can thus be achieved. The fluid film is in particular formed in an area between the two electrodes 13. SEBS can thus be in between the electrodes 13 as well.

The SEBS may be arranged such that an air pocket may be sealed off in the vicinity of the skin conductance electrodes, when the skin conductance electrodes contact the skin of the user. In this way, a hermetic sealing of a small area surrounding the skin conductance electrodes from the outside atmosphere is achieved. The air pocket is in particular formed in an area between the two electrodes.

The wearable sensor device 10 when worn by the user is pressed against the skin of the user, such that the edges of the wearable sensor device or the anti-slip element are pressed against the skin. The skin conductance electrodes 13 protrude from the holding element 11 by a distance, e.g. in the range between 50 to 1000 micrometer. More specifically, the distance can be between 100 and 700 micrometer.

The ring-shaped anti-slip element 14 protrudes from the holding element 11 as well, in particular to a larger extent than the at least one sensor. When pressed against the skin of the user, the anti-slip element 14 is slightly compressed so that both the anti-slip element 14 and the skin conductance electrodes 13 are in contact with the skin.

The size of the back plate 11 of the housing 12 is such that it fits almost all wrist sizes without compromising sensor signal quality. A small space is left between the anti-slip element 14 and the skin conductance electrodes 13 in order to help maintain the micro-climate and reduce the percentage of non-responders for which the sympathetic nervous system activity cannot be monitored.

The skin conductance electrodes 13 can be two separate elements separated by a certain distance, e.g. of at least 1.5mm. Alternatively, the two skin conductance electrodes 13 can be integrated in a single element, as long as they are isolated from each other.

The wearable sensor device 10 may further comprise a processing unit 16 configured to process the sensed parameter of the user, which is preferably arranged in the housing. Further elements like a communication unit and/or a display and/or a user interface may be provided as well.

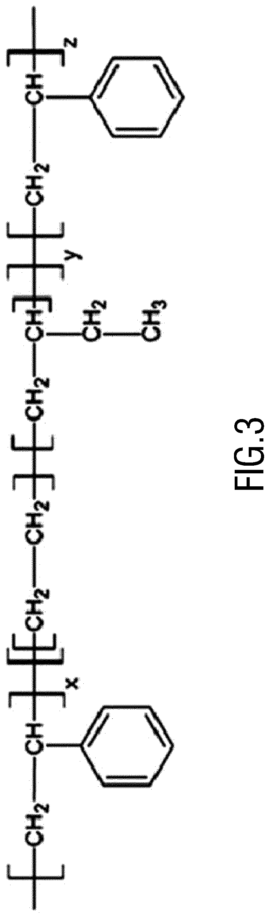

The polymeric material styrene ethylene butadiene styrene (SEBS) has the chemical formula shown in Fig. 3.

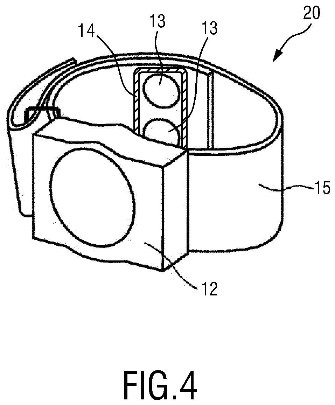

Fig. 4 shows a perspective view of a second embodiment of a wearable sensor device 20 according to the present invention. In this embodiment, the two skin conductance electrodes 13 are not arranged on the back plate 11 of the housing 12, but the fixation element 15 serves as holding element for holding the electrodes 13. The electrodes 13 may thus e.g. be arranged in or on the part of the fixation element 15 (in this example the wristband) that faces and contacts the skin, when it is fixed to the user's wrist. The electrodes 13 may e.g. be arranged in or on the part of the fixation element that is in contact with the underside of the wrist.

The anti-slip element 14 is arranged on the fixation element 15 as a closed ring around the electrodes 13. It may, in addition, also be arranged between the electrodes 13.

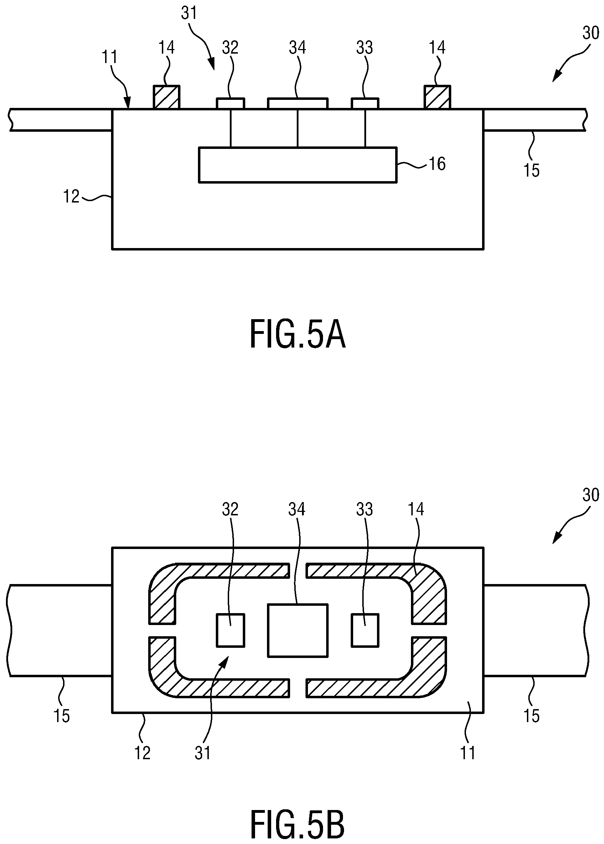

Fig. 5 shows a cross-sectional view (Fig. 5A) and a bottom view (Fig. 5B) of a third embodiment of the wearable sensor device 30 according to the present invention. In this embodiment, the wearable sensor 30 comprises a photoplethysmography sensor 31 including two LEDs 32, 33, one emitting red light and the other one emitting infrared light, and a photosensor 34 for sensing light reflected from the skin in response to the emitted red and infrared light. This enable measurement of vital signs like pulse rate and SpO2, as conventionally known in the field of vital signs monitoring using pulse oximeters.

In this embodiment the anti-slip element 14 is formed as an open ring with one or more opening so that sweat formed inside the ring can escape so that potential negative influences on the measurement can be avoided.

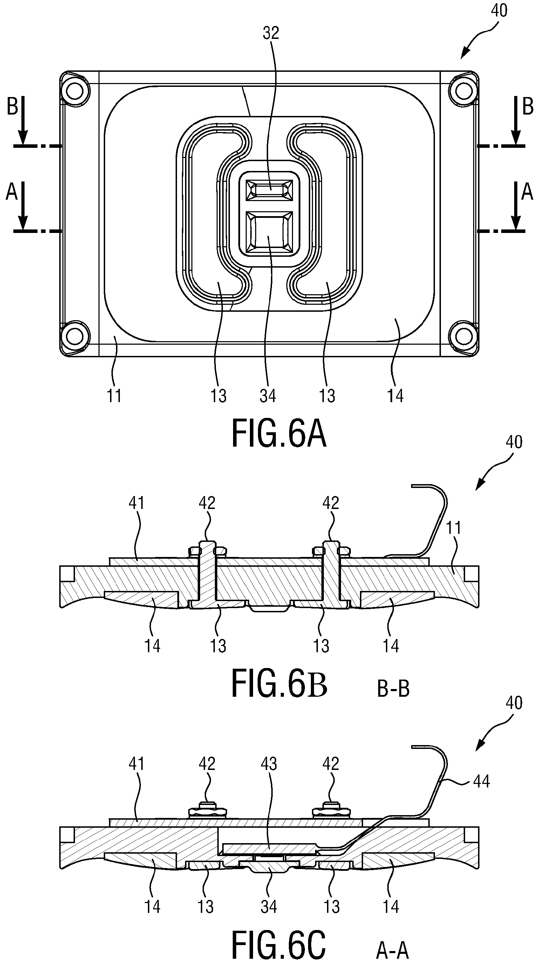

Fig. 6 shows a bottom view (Fig. 6A) and two cross-sectional views, one along cutting plane B-B (Fig. 6B) and another one along cutting plane A-A (Fig. 6C) of a fourth embodiment of a wearable sensor device 40 according to the present invention. Hereby, the housing 12 has been omitted.

In the wearable sensor device 40 both the skin conductance electrodes 13 and a photoplethysmography sensor comprising an LED 32 and a photosensor 34 are provided in combination. Fig. 6B particularly shows threaded metal cylinders 42, which go through holes in a first printed circuit board 41 carrying the skin conductance sensor electronics and which function as electrical connector as well as fixation. Fig. 6C particularly shows a second printed circuit board 43 carrying the photoplethysmograph sensor electronics and a flex foil interconnect 44 from the photoplethysmograph sensor printed circuit board 43 to a main printed circuit board (not shown).

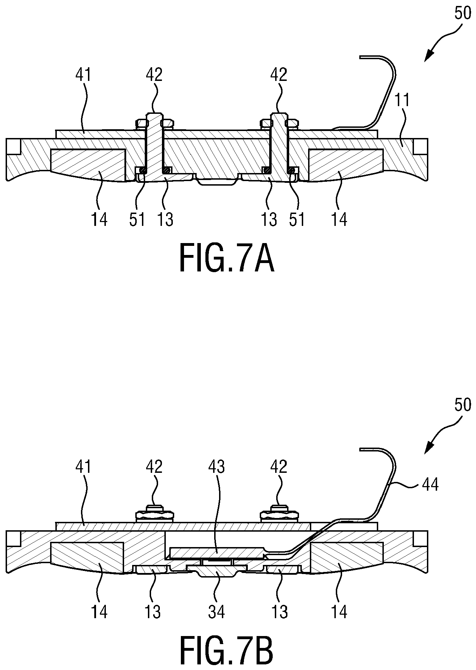

Fig. 7 shows two cross-sectional views of a fifth embodiment of a wearable sensor device 50 according to the present invention, one along cutting plane B-B (Fig. 7A) and another one along cutting plane A-A (Fig. 7B), i.e. along the same cutting planes as indicated in Fig. 6A.

A bottom view of the wearable sensor device 50 is identical to the bottom view shown in Fig. 6A for the wearable sensor device 40. Different from the wearable sensor device 40 the thickness of the anti-slip elements 14 is increased so that the anti-slip elements 14 (in thickness direction) lead through more than 50% of the thickness of the wearable sensor device 50 to make use of the elastic deformation of the anti-slip elements 14. Further, sealing rings 51 (O rings) are arranged around the threaded metal cylinders 42 at a position where the threaded metal cylinders 42 are connected to the electrodes 13.

In other embodiments of wearable sensor devices further and/or other sensor elements may be provided, such as an infrared thermometer.

In another embodiment no only a single ring of anti-slip material but several rings of anti-slip material may be formed around the at least one sensor element.

The present invention can generally be used in all kinds of wearable sensor devices and can e.g. be implemented as smartwatch, sensor patch, skin conductance sensor, or virtual reality headset.

The anti-slip elements 14 can e.g. be made by 3D printing or injection molding. As an alternative to SEBS, thermoplastic elastomers (TPEs) may be used, in particular injection moldable thermoplastic elastomers with a high friction coefficient with respect to human skin.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. Wearable sensor device (10, 20, 30) configured to be worn by a user for sensing a parameter of the user, the wearable sensor device comprising:
- a holding element (11, 15),
- at least one sensor element (13, 31) arranged in or on the holding element for contacting skin of the user and sensing the parameter of the user, and
- a ring-shaped anti-slip element (14) arranged on the holding element around the at least one sensor element, the anti-slip element protruding from the holding element and being made of styrene ethylene butadiene styrene.

2. Wearable sensor device as claimed in claim 1,
wherein the ring-shaped anti-slip element (14) is formed as closed ring.

3. Wearable sensor device as claimed in claim 1,
wherein the ring-shaped anti-slip element (14) is formed as open ring comprising two or more ring sections with openings in between.

4. Wearable sensor device as claimed in any one of the preceding claims,
wherein the at least one sensor (13, 31) and the ring-shaped anti-slip element (14) protrude from the holding element (11, 15), wherein the ring-shaped anti-slip element (14) protrudes from the holding element to a larger extent than the at least one sensor.

5. Wearable sensor device as claimed in any one of the preceding claims,
wherein the styrene ethylene butadiene styrene has a low hardness, in particular hardness below 30 Shore A.

6. Wearable sensor device as claimed in any one of the preceding claims,
further comprising a fixation element (15) for fixing the wearable sensor device to a body part of the user.

7. Wearable sensor device as claimed in claim 6, wherein the holding element is part of the fixation element (15).

8. Wearable sensor device as claimed in any one of the preceding claims, further comprising a housing (12).

9. Wearable sensor device as claimed in claim 8, wherein the holding element is a back plate (11) of the housing (12).

10. Wearable sensor device as claimed in any one of the preceding claims,
comprising at least two skin conductance electrodes (14) and/or a plethysmography sensor (31) including a light source (32, 33) and a photo detector (34) and/or an infrared thermometer.

11. Wearable sensor device as claimed in any one of the preceding claims,
wherein the ring-shaped anti-slip element (14) is arranged such that an air pocket is sealed off in the vicinity of the at least one sensor (13, 31), when the at least one sensor contacts the skin of the user.

12. Wearable sensor device as claimed in any one of the preceding claims,
wherein the at least one sensor (13, 31) protrudes between 50 to 2000 micrometer from the holding element (11), in particular between 100 and 700 micrometer.

13. The wearable device as claimed in any one of the preceding claims,
further comprising a processing unit (16) configured to process the sensed parameter of the user.

14. The wearable device of claim 8 and 11,
wherein the processing unit (16) is at least partly arranged in a housing (12).

15. The wearable device as claimed in any one of the preceding claims,
wherein the styrene ethylene butadiene styrene has an elasticity that is sufficient to follow the shape of the wrist of the user.
